# EUROPÄISCHE PATENTANMELDUNG

(11) **EP 0 923 916 A1**
(43) Veröffentlichungstag der Anmeldung: **23.06.1999**
(21) Anmeldenummer: 97811002.1
(22) Anmeldetag: 19.12.1997
(51) Int. Cl.: A61F 2/38

(54) **Kniegelenkprothese**

(71) Anmelder: Sulzer Orthopädie AG, 6340 Baar (CH)
(72) Erfinder: Schwägerl, Wolfgang, Prof. Dr. med., 1160 Wien (AT); Böhler, Nikolaus, Prof. Dr. med., 4020 Linz (AT); Brack, René, 6330 Cham (CH); Frei, Heribert, 8200 Schaffhausen (CH); Gyssler, Bernhard, 8810 Horgen (CH)
(74) Vertreter: Heinen, Detlef

(57) **Zusammenfassung**

Die Kniegelenkprothese umfasst ein Tibiateil (1), ein Femurteil (2) mit zwei Kondylen (21), sowie zwei auf dem Tibiateil (1) befindliche, unbeweglich angeordnete Lagerflächen (31). Ferner umfasst sie ein Stabilisierungsstück (41), welches zwischen die beiden Kondylen (21) des Femurteils (2) hineinragt und dort mit entsprechenden am Femurteil (2) vorgesehenen Stabilisierungsflächen (210) zusammenwirkt. Das Stabilisierungsstück (41) ist an einem separaten Zapfen (4) vorgesehen, welcher zwischen den beiden Lagerflächen (31) in eine Bohrung (15) im Tibiateil (1) einbringbar ist und welcher drehbar gelagert ist.

## Beschreibung

Die Erfindung betrifft eine Kniegelenkprothese gemäss dem Oberbegriff des unabhängigen Patentanspruchs.

Kniegelenkprothesen stehen heutzutage in einer grossen Anzahl verschiedener Ausführungsformen zur Verfügung. Ihre Funktionsprinzipien unterscheiden sich zum Teil ganz erheblich, was unter anderem davon abhängt, dass bei den Patienten recht unterschiedliche Verhältnisse gegeben sind. Beispielsweise können noch alle Bänder erhalten und funktionstüchtig sein, es kann aber auch nur noch das eine Kreuzband oder es können nur noch die Seitenbänder funktionstüchtig sein, oder es können die Seitenbänder oder sogar alle Bänder fehlen oder nicht mehr funktionstüchtig sein.

Insbesondere bei solchen Patienten, bei denen gar keine Bänder mehr vorhanden sind oder bei denen die Bänder nicht mehr oder nicht mehr voll funktionstüchtig sind, muss eine entsprechende Prothese gewählt werden, die dies berücksichtigt. Eine derartige Kniegelenkprothese ist beispielsweise aus der DE-A-29 33 124 bekannt. Die dort beschriebene Kniegelenkprothese umfasst ein Tibiateil und einen darauf (Z.B. durch Kleben) befestigten Lagerkörper oder zwei einzelne darauf befestigte Lagerschalen (Lagerflächen), üblicherweise als Meniskusteil(e) bezeichnet. Der Lagerkörper (bzw. die einzelnen Lagerschalen) ist (sind) dadurch relativ zum Tibiateil unbeweglich angeordnet. Weiterhin umfasst die dort beschriebene Kniegelenkprothese ein Femurteil mit zwei Kondylen (Laufflächen). Zwischen die beiden Kondylen ragt ein Stabilisierungsstück hinein, welches mit entsprechenden Stabilisierungsflächen am Femurteil zusammenwirkt und für eine Varus/Valgus-Stabilisierung sorgt. Das Stabilisierungsstück ist dabei am Tibiateil angeformt und ist somit integraler Bestandteil des Tibiateils.

Eine derartige Prothese ist absolut funktionstüchtig. Jedoch weist sie den Nachteil auf, dass eine Drehung der Kondylen und damit des Femurteils relativ zum Tibiateil und dem darauf befindlichen Meniskusteil bzw. den einzelnen Lagerschalen (Lagerflächen) unmöglich ist. Das liegt daran, dass das am Tibiateil angeformte Stabilsierungsstück, welches zwischen die Kondylen hineinragt und mit den Stabilisierungsflächen am Femurteil zusammenwirkt, eine solche Drehung verhindert, was wiederum die Beweglichkeit einschränkt. Darüberhinaus ist es umittelbar einleuchtend, dass eine derartige Prothese, insbesondere natürlich das Tibiateil mit dem daran angeformten Stabilisierungsstück, von der Herstellungsseite her mit recht erheblichem Aufwand verbunden ist.

Es ist daher eine Aufgabe der Erfindung, eine Prothese vorzuschlagen, die die positiven Eigenschaften der eingangs genannten Prothese beibehält, die aber doch eine Drehung des Femurteils relativ zum Tibiateil bzw. relativ zum Lagerkörper grundsätzlich erlaubt. Gleichzeitig soll die Prothese von der Herstellungsseite her möglichst wenig aufwendig sein.

Diese Aufgabe wird durch die Erfindung, wie sie im unabhängigen Patentanspruch charakterisiert ist, gelöst. Erfindungsgemäss ist das Stabilisierungsstück an einem separaten Zapfen vorgesehen, welcher zwischen den beiden Lagerflächen in eine Bohrung im Tibiateil einbringbar ist und welcher drehbar gelagert ist. Dies ermöglicht, dass das Femurteil relativ zum Tibiateil bzw. relativ zu den Lagerflächen drehbar ist. Darüberhinaus ist die Herstellung der einzelnen Teile vergleichsweise einfach, da es wesentlich einfacher ist, ein Tibiateil ohne Stabilisierungsstück und einen gesonderten Zapfen herzustellen und diesen beim Zusammensetzen der Prothese in eine Bohrung im Tibiateil einzubringen, als ein Tibiateil herzustellen, an welchem der Zapfen als integraler Bestandteil angeformt ist. Auch das Zusammensetzen der Prothese ist einfach durchzuführen.

In einem vorteilhaften Ausführungsbeispiel sind die beiden Lagerflächen an einem gemeinsamen Lagerkörper - einem Meniskusteil - vorgesehen. Der gemeinsame Lagerkörper weist eine Öffnung auf, durch die hindurch der Zapfen in die Bohrung im Tibiateil einbringbar ist. Somit muss jeweils nur ein einziger, gemeinsamer Lagerkörper hergestellt werden.

In einem weiteren vorteilhaften Ausführungsbeispiel der erfindungsgemässen Kniegelenkprothese ist im Stabilisierungsstück des Zapfens eine nach unten offene Nut vorgesehen. Das Femurteil ist mit einem die Stabilisierungsflächen verbindenden Steg versehen, der in diese Nut hineinragt. Dieses Ausführungsbeispiel ist insofern vorteilhaft, als dadurch ein anteriorer wie auch posteriorer Anschlag für den Steg vorgesehen ist, sodass eine Luxation (Ausrenkung) des Gelenks nicht möglich ist. Gleichzeitig bleibt natürlich die Varus/Valgus-Stabilisierung erhalten, weil nach wie vor das Stabilisierungsstück des Zapfens mit den entsprechenden Stabilisierungsflächen am Femurteil zusammenwirkt.

In einem weiteren Ausführungsbeispiel der erfindungsgemässen Kniegelenkprothese ist der Zafpen mit einem umlaufenden Vorsprung versehen, welcher beim Einbringen des Zapfens durch die Öffnung des Lagerkörpers über einen entsprechenden Vorsprung am Lagerkörper gleitet und somit anschliessend ein Herausgleiten des Zapfens durch die Öffnung im Lagerkörper verhindert. Ein Herausgleiten des Zapfens und eine dadurch hervorgerufene Luxation der Prothese oder eine Dislokation von einzelnen Teilen der Prothese ist somit nicht möglich, weil ein selbständiges Herausgleiten des Zapfens zuverlässig verhindert wird. Gleichzeitig ist aber der Zapfen in axialer Richtung (also in distaler/proximaler) Richtung der Prothese so lange beweglich, bis der umlaufende Vorsprung am Zapfen gegen den entsprechenden Vorsprung am Lagerkörper stösst. Eine Beweglichkeit der Prothese in axialer Richtung ist dadurch innerhalb gewisser Grenzen gewährleistet.

Dennoch ist es natürlich möglich, den Zapfen unter entsprechendem Kraftaufwand auch wieder zu entfernen, was für den Fall, dass die Prothese ersetzt werden muss, auch wünschenswert ist.

In einem weiteren Ausführungsbeispiel der erfindungsgemässen Kniegelenkprothese sind am Lagerkörper und am Tibiateil Mittel zum Befestigen des Lagerkörpers am Tibiateil vorgesehen. Dies ermöglicht eine getrennte Herstellung von Tibiateil und Lagerkörper, was zum einen die Herstellung vereinfacht, zum anderen kann die Prothese nach den individuellen Gegebenheiten bei den einzelnen Patienten noch im Operationssaal zusammengestellt werden, was eine Versorgung des Patienten mit der für ihn optimalen Prothese gewährleistet. Zum Beispiel kann es für verschiedene Patienten erforderlich sein, ein gleichartiges Tibiateil, aber ein unterschiedlich dickes Meniskusteil zu verwenden. Dies ist hierdurch auf einfache Weise möglich.

Insbesondere können die Mittel zum Befestigen des Lagerkörpers Vorsprünge sein, die sowohl am Tibiateil als auch am Lagerkörper vorgesehen sind, die sich bei befestigtem Lagerkörper gegenseitig hintergreifen, nach dem Prinzip eines Schnappverschlusses. Auf diese Weise kann der Lagerkörper auf einfache Art und Weise mit dem Tibiateil verbunden werden und ist relativ zu diesem unbeweglich angeordnet. Auch von der Herstellungsseite betrachtet ist diese Variante sehr einfach.

In einem weiteren Ausführungsbeispiel der erfindungsgemässen Kniegelenkprothese weist das Tibiateil einen Verankerungsabschnitt auf, der mit einem separaten Verankerungsschaft verbindbar ist. In wieder einem weiteren Ausführungsbeispiel der erfindungsgemässen Kniegelenkprothese weist das Femurteil einen Verankerungsabschnitt auf, der mit einem separaten Verankerungsschaft verbindbar ist. Diese Massnahmen erhöhen ebenfalls die Flexibilität des Operateurs, der nötigenfalls noch im Operationssaal die für den Patienten optimale Länge des Verankerungsschafts auswählen kann, weil die Prothese praktisch modular zusammensetzbar ist.

Im folgenden wird die Erfindung anhand der Zeichnung näher erläutert. Dabei zeigen, teilweise schematisch und/oder im Schnitt:
- Fig. 1: ein Ausführungsbeispiel der erfindungsgemässen Kniegelenkprothese in Explosionsdarstellung,
- Fig. 2: eine Seitenansicht des Zapfens des Ausführungsbeispiels der Kniegelenkprothese gemäss Fig. 1,
- Fig. 3: einen Längsschnitt durch den Zapfen,
- Fig. 4: die Einzelheit IV der Fig. 3,
- Fig. 5: eine Aufsicht auf das Tibiateil des Ausführungsbeispiels der Kniegelenkprothese gemäss Fig. 1,
- Fig. 6: die Einzelheit VI der Fig. 5 (anteriorer Vorsprung),
- Fig. 7: die Einzelheit VII der Fig. 5 (posteriorer Vorsprung)
- Fig. 8: das Meniskusteil des Ausführungsbeispiels der Kniegelenkprothese gemäss Fig. 1,
- Fig. 9: die Einzelheit IX der Fig. 8 (anteriorer Vorsprung)
und
- Fig. 10: die Einzelheit X der Fig. 8 (posteriorer Vorsprung).

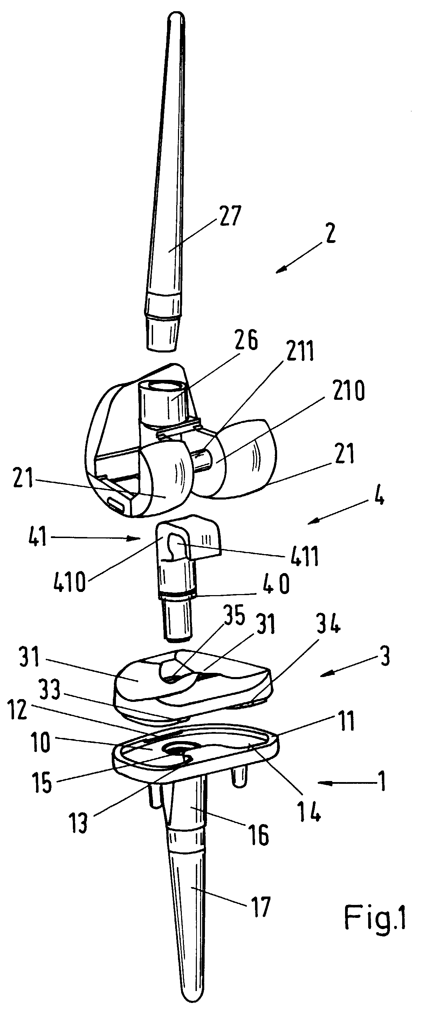

Bei dem in Fig. 1 dargestellten Ausführungsbeispiel der erfindungsgemässen Kniegelenkprothese erkennt man ein Tibiateil 1, ein Femurteil 2, einen gemeinsamen Lagerkörper in Form eines Meniskusteils 3, sowie einen Zapfen 4. Anstelle des Meniskuksteils 3 können prinzipiell auch zwei separate Lagerschalen mit entsprechenden Lagerflächen vorgesehen sein.

Das Tibiateil 1 weist auf seiner dem Femurteil 2 bzw. dem Meniskusteil 3 zugewandten Fläche (Tibiaplateau) eine grossflächige Aussparung 10 auf, sodass ein umlaufender Rand 11 vorgesehen ist. An diesem umlaufenden Rand 11 sind Mittel zum Befestigen des Meniskusteils 3 vorgesehen. Dies sind der nach innen weisende Vorsprung 12 im vorderen Bereich des umlaufenden Randes 11, sowie entsprechende nach innen weisende Vorsprünge im hinteren Bereich, die in Fig. 1 mit den Bezugszeichen 13 und 14 bezeichnet sind.

Am Meniskusteil 3 sind entsprechende Vorsprünge vorgesehen, wobei in Fig. 1 nur die Vorsprünge 33 und 34 zu erkennen sind. Zur Befestigung des Meniskusteils 3 am Tibiateil 1 wird nun das Meniskusteil 3 in die Aussparung 10 hineingeführt. Dies erfolgt z.B. derart, dass die Vorsprünge 33,34 des Meniskusteils 3 und die entsprechenden Vorsprünge 13,14 des Tibiateils ineinander greifen und dann der Vorsprung 32 am Meniskusteil 3 über den Vorsprung 12 am Tibiateil 1 gleitet, wodurch das Meniskusteil 3 nach Art eines Schnappverschlusses einschnappt. Somit ist das Meniskusteil 3 mit dem Tibiateil 1 fest verbunden, also relativ zum Tibiateil 1 unbeweglich.

Das Meniskusteil 3 weist eine Öffnung 35 in Form einer Durchgangsbohrung auf. Durch diese Öffnung 35 hindurch kann der Zapfen 4 in eine Bohrung 15 im Tibiateil 1 eingebracht werden. Der Zapfen 4 ist mit einem umlaufenden Vorsprung 40 versehen, welcher beim Einbringen des Zapfens 4 durch die Öffnung 35 des Meniskusteils 3 über einen entsprechenden Vorsprung 36 am Meniskusteil 3 gleitet, sodass anschliessend ein unbeabsichtigtes Herausgleiten des Zapfens 4 durch die Öffnung 35 nicht mehr möglich ist.

Ferner weist der Zapfen 4 ein Stabilisierungsstück 41 auf, welches hier dem oberen Bereich des Zapfens entspricht. Dieses Stabilisierungsstück 41 und insbesondere dessen Seitenflächen 410 bewirken im Zusammenhang mit den Stabilisierungsflächen 210 am Femurteil 2 eine Varus/Valgus-Stabilisierung.

Weiterhin ist im Stabilisierungsstück 41 des Zapfens 4 eine nach unten offene Nut 411 vorgesehen. Ist die Prothese zusammengesetzt, greift in diese Nut 411 am Stabilisierungsstück 41 des Zapfens 4 ein Steg 211 ein, der die Stabilisierungsflächen 210 des Femurteils 2 miteinander-verbindet. Der Steg 211 erstreckt sich also in der interkondylären Nische bzw. im Falle eines interkondylären Kastens im interkondylären Kasten, in welchem er intergriert sein kann. Auf diese Art und Weise ist ein anteriorer und ein posteriorer Anschlag definiert, sodass ein unerwünschtes Abgleiten des Femurteils 2 in anteriorer oder posteriorer Richtung verhindert wird. Gleichzeitig ist eine gewisse Flexibilität des Femurteils 2 relativ zum Tibiateil 1 bzw. relativ zum Meniskusteil 3 in axialer Richtung gewährleistet, weil der Zapfen 4 ja in axialer Richtung so lange beweglich ist, bis der Vorsprung 40 am Zapfen 4 an dem entsprechenden Vorsprung des Meniskusteils 3 anschlägt, damit der Zapfen 4 nicht unbeabsichtigt durch die Öffnung 35 herausgleiten kann.

Es ist also einerseits ein unbeabsichtigtes Herausgleiten des Zapfens 4 zuverlässig vermieden, gleichzeitig ist eine Flexibilität des Femurteils 2 relativ zum Tibiateil 1 bzw. relativ zum Meniskusteil 3 in axialer Richtung gegeben. Da der Zapfen 4 in der Bohrung 15 des Tibiateils 1 drehbar gelagert ist und sich der Zapfen 4 auch in der Öffnung 35 des Meniskusteils 3 drehen kann, ist im Zustand der Flexion eine gewisse Drehung des Femurteils 2 relativ zum Tibiateil 1 bzw. relativ zum Meniskusteil 3 möglich. Im Zustand der Extension ist eine solche Drehung des Femurteils 2 relativ zum Tibiateil 1 bzw. relativ zum Meniskusteil 3 aufgrund der hohen Kongruenz der Kondylen 21 und der Lagerflächen 31 des Meniskusteils 3 nicht möglich.

Es soll noch darauf hingewiesen werden, dass das Tibiateil 1 und auch das Femurteil 2 Ansatzstücke 16 bzw. 26 aufweisen, welche jeweils mit einem Verankerungsschaft 17 bzw. 27 verbindbar ist, beispielsweise mittels einer konischen Verbindung. Dadurch wird erreicht, dass notfalls sogar noch während der Operation, im Normalfall aber bereits davor, die für den jeweiligen Patienten optimalen Komponenten praktisch nach Art eines Bausatzes zusamnengestellt werden können, somit der Patient mit der optimal auf ihn zugeschnittenen Prothese versorgt werden kann.

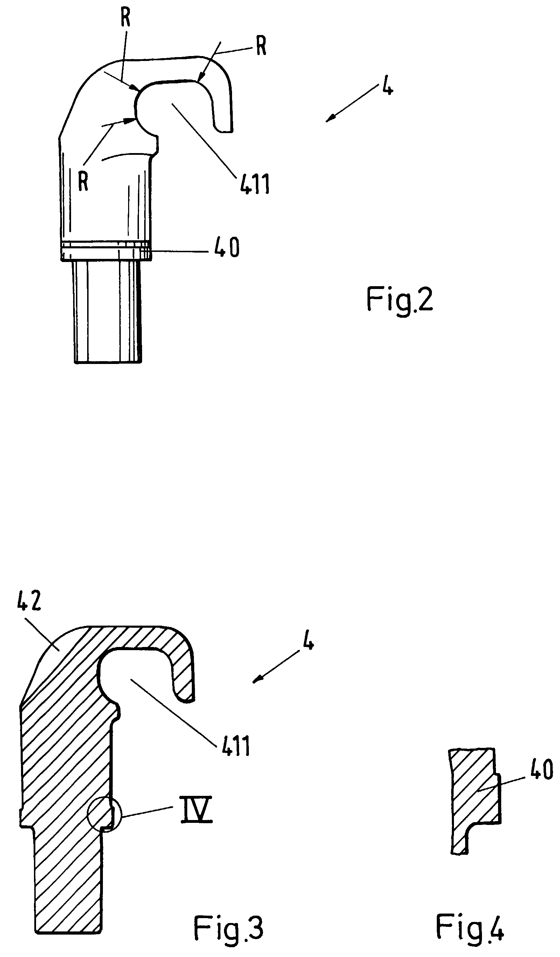

Fig. 2 zeigt eine Seitenansicht des Zapfens 4, aus welcher die Kontur der nach unten offenen Nut 411 gut erkennbar ist. Die Nut weist an verschiedenen Stellen einen Radius R auf, der etwa dem Radius des Stegs 211 (Fig.1) entspricht, welcher die beiden Kondylen verbindet. Dies hat beim Beugen des Knies eine hohe Kongruenz zur Folge, was die Flächenpressung (Druck) verringert und somit einen geringen Flexionswiderstand zur Folge hat, vereinfacht gesprochen lässt sich das Knie vergleichsweise leicht beugen.

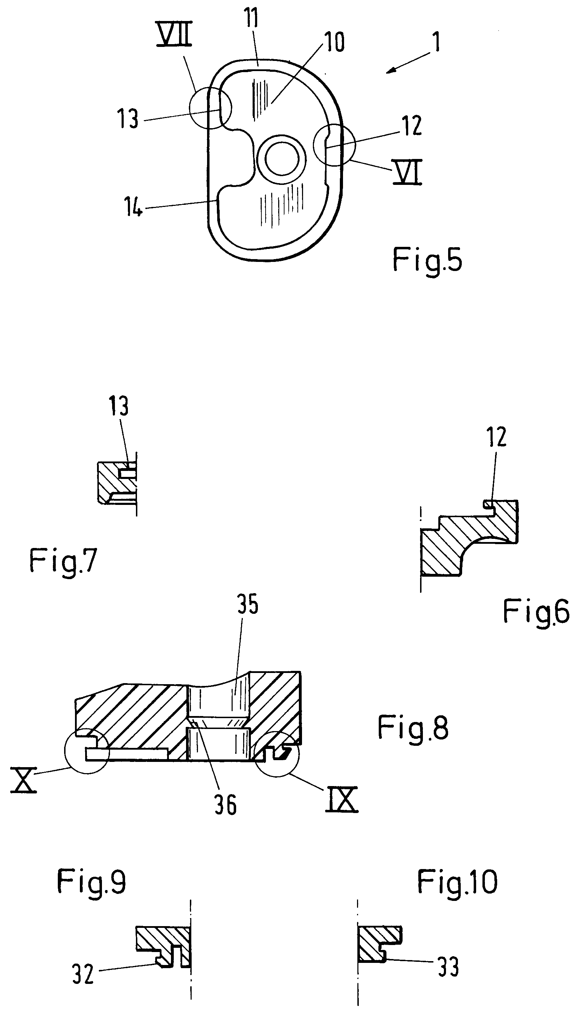

Fig. 3 zeigt einen Längsschnitt durch den Zapfen 4, aus welchem erkennbar ist, dass er eine Grube 42 für die Patella (Kniescheibe) aufweist, in welcher diese beim Beugen und Strecken des Knies gleiten kann. Ferner ist in Fig. 3 noch die Einzelheit IV angedeutet, die in Fig. 4 vergrössert dargestellt ist.

In Fig. 4 erkennt man diese Einzelheit, bei der es sich um den bereits früher erwähnten umlaufenden Vorsprung 40 handelt, der im Zusammenwirken mit einem entsprechenden Vorsprung 36 am Meniskusteil 3 derart zusammenwirkt, dass ein unbeabsichtigtes Herausgleiten des Zapfens 4 durch die Öffnung 35 des Meniskusteils 3 vermieden wird.

Fig. 5 zeigt eine Aufsicht auf das Tibiateil 1, aus der noch einmal die Vertiefung 10, der umlaufende Rand 11 sowie die Vorsprünge 12,13,14 erkennbar sind. Besser erkennbar ist der anteriore Vorsprung 12 noch in Fig. 6, welche eine Schnittdarstellung der Einzelheit VI, also einen Schnitt durch diesen Vorsprung 12, zeigt. Fig. 7 zeigt eine entsprechende Schnittdarstellung der Einzelheit VII, also eine Schnittdarstellung des posterioren Vorsprungs 13, der posteriore Vorsprung 14 entspricht diesem Vorsprung 13.

Fig. 8 zeigt einen Schnitt durch das Meniskusteil 3 mit den Einzelheiten IX und X, wobei die Einzelheit IX, also der Vorsprung 32, in Fig. 9 und die Einzelheit X, also der Vorsprung 33 (der Vorsprung 34 ist analog ausgebildet) in Fig. 10 noch einmal vergrössert dargestellt sind. Das Meniskusteil 3 bzw. dessen Vorsprünge 33,34 könnnen hinter die entsprechenden Vorsprünge 13,13 am Tibiateil 1 gleiten und anschliessend kann der Vorsprung 32 des Meniskusteils 3 über den Vorsprung 12 am Tibiateil 1 gleiten und einschnappen. Das Meniskusteil ist dann unbeweglich mit dem Tibiateil 1 verbunden.

Die Lagerflächen 31 des Meniskusteils 3 sind, wie bereits erwähnt, so ausgebildet, dass im Zustand der Extension, also bei gestrecktem Knie, eine hohe Kongruenz von Lagerflächen 31 und den Kondylen 21 des Femurteils 2 besteht. Eine Drehung des Femurteils 2 relativ zum Tibiateil 1 bzw. relativ zum Meniskusteil 3 ist in diesem Zustand im Prinzip nicht möglich. Im Zustand der Flexion hingegen, also bei gebeugtem Knie, erlaubt die geringere Kongruenz eine Drehung des Femurteils 2 relativ zum Tibiateil 1 bzw. zum Meniskusteil 3, weil der Zapfen 4 drehbar gelagert ist. Gleichzeitig bleibt die Varus/Valgus-Stabilisierung erhalten.

## Patentansprüche

1. Kniegelenkprothese mit einem Tibiateil (1), einem Femurteil (2) mit zwei Kondylen (21), sowie mit zwei auf dem Tibiateil (1) befindlichen, unbeweglich angeordneten Lagerflächen (31), und mit einem Stabilisierungsstück (41), welches zwischen die beiden Kondylen (21) des Femurteils (2) hineinragt und dort mit entsprechenden am Femurteil (2) vorgesehenen Stabilisierungsflächen (210) zusammenwirkt, dadurch gekennzeichnet, dass das Stabilisierungsstück (41) an einem separaten Zapfen (4) vorgesehen ist, welcher zwischen den beiden Lagerflächen (31) in eine Bohrung (15) im Tibiateil (1) einbringbar ist und welcher drehbar gelagert ist.

2. Kniegelenkprothese nach Anspruch 1, dadurch gekennzeichnet, dass die beiden Lagerflächen (31) an einem gemeinsamen Lagerkörper (3) vorgesehen sind, und dass dieser Lagerkörper (3) eine Öffnung (35) aufweist, durch die hindurch der Zapfen (4) in die Bohrung (15) des Tibiateils (1) einbringbar ist.

3. Kniegelenkprothese nach Anspruch 1 oder 2, dadurch gekennzeichnet, dass im Stabilisierungsstück (41) des Zapfens (4) eine nach unten offene Nut (411) vorgesehen ist, und dass das Femurteil (2) mit einem die Stabilisierungsflächen (210) verbindenden Steg (211) versehen ist, der in diese Nut (411) hineinragt.

4. Kniegelenkprothese nach Anspruch 2 oder 3, dadurch gekennzeichnet, dass der Zafpen (4) mit einem umlaufenden Vorsprung (40) versehen ist, welcher beim Einbringen des Zapfens (4) durch die Öffnung (35) des Lagerkörpers (3) über einen entsprechenden Vorsprung (36) am Lagerkörper (3) gleitet und somit anschliessend ein Herausgleiten des Zapfens (4) durch die Öffnung (35) im Lagerkörper (3) verhindert.

5. Kniegelenkprothese nach einem der Ansprüche 1 bis 4 dadurch gekennzeichnet, dass am Lagerkörper (3) und am Tibiateil (1) Mittel zum Befestigen des Lagerkörpers (3) am Tibiateil (1) vorgesehen sind.

6. Kniegelenkprothese nach Anspruch 5, dadurch gekennzeichnet, dass die Mittel zum Befestigen des Lagerkörpers Vorsprünge (12,13,14,32,33,34) sind, die sowohl am Tibiateil (1) als auch am Lagerkörper (3) vorgesehen sind, die sich bei befestigtem Lagerkörper (3) gegenseitig hintergreifen.

7. Kniegelenkprothese nach einem der vorangehenden Ansprüche, dadurch gekennzeichnet, dass das Tibiateil (1) einen Verankerungsabschnitt (16) aufweist, der mit einem separaten Verankerungsschaft (17) verbindbar ist.

8. Kniegelenkprothese nach einem der vorangehenden Ansprüche, dadurch gekennzeichnet, dass das Femurteil (2) einen Verankerungsabschnitt (26) aufweist, der mit einem separaten Verankerungsschaft (27) verbindbar ist.
